(19) Europäisches Patentamt
European Patent Office
Office européen des brevets

(11) Publication number: **0 190 010 B1**

# (12) EUROPEAN PATENT SPECIFICATION

(45) Date of publication of patent specification: **10.04.91** (51) Int. Cl.[5]: **A61K 7/075**

(21) Application number: **86300447.9**

(22) Date of filing: **23.01.86**

The file contains technical information submitted after the application was filed and not included in this specification

## (54) Shampoo compositions and method.

(30) Priority: **25.01.85 US 694870**

(43) Date of publication of application: **06.08.86 Bulletin 86/32**

(45) Publication of the grant of the patent: **10.04.91 Bulletin 91/15**

(84) Designated Contracting States: **AT BE CH DE FR IT LI LU NL SE**

(56) References cited:
**EP-A- 0 074 264**
**EP-A- 0 113 418**
**GB-A- 2 142 348**
**US-A- 3 562 786**

(73) Proprietor: **THE PROCTER & GAMBLE COMPANY**
**One Procter & Gamble Plaza**
**Cincinnati Ohio 45202(US)**

(72) Inventor: **Bolich, Raymond Edward, Jr.**
**7201 Striker Road**
**Maineville Ohio 45039(US)**
Inventor: **Williams, Theresa Bakken**
**2536 Briarcliffe Avenue**
**Cincinnati Ohio 45212(US)**

(74) Representative: **Brooks, Maxim Courtney et al**
**Procter & Gamble (NTC) Limited Whitley Road Longbenton**
**Newcastle-upon-Tyne NE12 9TS(GB)**

EP 0 190 010 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention is related to conditioning shampoos which have a dispersed, insoluble, non-volatile silicone phase and are stabilized through the use of xanthan gum.

Human hair becomes soiled due to its contact with the surrounding atmosphere and, to a greater extent, from sebum secreted by the head. The build-up of the sebum causes the hair to have a dirty feel and an unattractive appearance. The soiling of the hair necessitates it being shampooed with frequent regularity.

Shampooing the hair cleans by removing excess soil and sebum. However, the shampooing process has disadvantages in that the hair is left in a wet, tangled and generally unmanageable state. A variety of approaches have been developed to alleviate the after-shampoo problems. These range from the inclusion of hair conditioning aids in shampoos to post-shampoo application of hair conditioners, i.e., hair rinses. Hair rinses typically work by depositing a polymeric film or other material onto the hair. However, such solutions to a very prevalent problem have not been fully satisfactory. For one thing, hair rinses are generally liquid in nature and must be applied in a separate step following the shampooing, left on the hair for a length of time, and rinsed with fresh water. This, of course, is time consuming and is not convenient.

While shampoos have been disclosed which contain conditioning aids, they have not been totally satisfactory for a variety of reasons. One problem relates to compatibility problems between good cleaning anionic surfactants and the fatty cationic agents which are good conditioning agents. This has caused other surfactants such as nonionics, amphoterics and zwitterionics to be examined by workers in the field. Many of these efforts are reflected in patents issued in the conditioning shampoo area. See for example US-A-3,849,348 , US-A-3,990,991 , and US-A-3,822,312 .

The use of these other surfactants solved many of the compatibility problems but still did not provide complete answers in all areas. For instance cationic conditioners may not deliver the desired level of softness desired by users. Materials which can provide increased softness are silicones, both those which are soluble as well as insoluble in the shampoo matrix.

Silicones in shampoo compositions have been disclosed in a number of different publications. Such publications include US-A-2,826,551 , US-A-3,964,500 , US-A-4,364,837 , GB-A-O,849,433 , US-A-4,341,799 , and US-A-4,465,619 . While these patents disclose silicone containing compositions, they also do not provide answers to all of the problems encountered in making a totally satisfactory product. One unsolved problem is that of keeping a dispersed, insoluble, non-volatile silicone material suspended and the total product stable while still providing satisfactory shampoo performance. A variety of materials have been included in silicone containing shampoos for purposes of thickening and stabilization but totally satisfactory solutions are lacking. It has been surprisingly found that compositions comprising specific components can provide stable compositions without interfering unduly with the deposit of the silicone material onto the hair and other shampoo functions.

It is an object of the present invention to provide a stable silicone containing conditioning shampoo.

It is a further object of the present invention to provide silicone shampoo compositions containing xanthan gum.

It is a further object of the present invention to provide shampoos which provide good conditioning (e.g., ease of combing, softness, feel, etc.) to hair.

It is a further object of the present invention to provide an improved method of shampooing and conditioning hair.

These and other objects will become readily apparent from the detailed description which follows.

Unless otherwise indicated, all percentages and ratios herein are by weight.

According to the present invention, there is provided a shampoo composition comprising:

(a) from 5% to 50% by weight of synthetic anionic surfactant or a mixture of synthetic anionic surfactants;

(b) from 0.1% to 10% by weight of a dispersed, insoluble, non-volatile silicone or a mixture of dispersed, insoluble, non-volatile silicones; and

(c) water;

characterised in that the composition additionally comprises from 0.4% to 5% by weight of xanthan gum.

The essential and optional components of the present invention are given in the following paragraphs.

### Surfactant

An essential component of the present compositions is a synthetic anionic surfactant. The surfactant is present at a level of from 5% to 50%, preferably from 10% to 30%.

Synthetic anionic detergents useful herein are alkyl and alkyl ether sulfates. These materials have the respective formulae $ROSO_3M$ and $RO(C_2H_4O)_xSO_3M$ wherein R is alkyl or alkenyl of about 10 to about 20 carbon atoms, x is 1 to 10, and M is a water-soluble cation such as ammonium, sodium, potassium and triethanolamine. The alkyl ether sulfates useful in the present invention are condensation products of ethylene oxide and monohydric alcohols having about 10 to about 20 carbon atoms. Preferably, R has 14 to 18 carbon atoms in both the alkyl and alkyl ether sulfates. The alcohols can be derived from fats, e.g., coconut oil or tallow, or can be synthetic. Lauryl alcohol and straight chain alcohols derived from coconut oil are preferred herein. Such alcohols are reacted with 1 to 10, and especially 3, molar proportions of ethylene oxide and the resulting mixture of molecular species, having, for example, an average of 3 moles of ethylene oxide per mole of alcohol, is sulfated and neutralized.

Specific examples of alkyl ether sulfates of the present invention are sodium coconut alkyl triethylene glycol ether sulfate; lithium tallow alkyl triethylene glycol ether sulfate; and sodium tallow alkyl hexaoxyethylene sulfate. Highly preferred alkyl ether sulphates are those comprising a mixture of individual compounds, said mixture having an average alkyl chain length of from about 12 to 16 carbon atoms and an average degree of ethoxylation of from about 1 to 4 moles of ethylene oxide. Such a mixture also comprises from about 0 to 20% by weight $C_{12-13}$ compounds; from 60 to 100% by weight of $C_{14-15-16}$ compounds, from about 0 to 20% by weight of $C_{17-18-19}$ compounds; from about 3 to 30% by weight of compounds having a degree of ethoxylation of 0; from about 45 to 90% by weight of compounds having a degree of ethoxylation of from 1 to 4; from about 10 to 25% by weight of compounds having a degree of ethoxylation of from 4 to 8; and from about 0.1 to 15% by weight of compounds having a degree of ethoxylation greater than 8.

Additional examples of anionic synthetic detergents which come within the terms of the present invention are the reaction product of fatty acids esterified with isethionic acid and neutralized with sodium hydroxide where, for example, the fatty acids are derived from coconut oil; sodium or potassium salts of fatty acid amides of methyl tauride in which the fatty acids, or example, are derived from coconut oil. Other anionic synthetic detergents of this variety are set forth in US-A-2,486,921; US-A-2,486,922; and US-A-2,396,278.

Still other anionic synthetic detergents include the class designated as succinamates. This class includes such surface active agents as disodium N-octadecylsulfosuccinamate; tetrasodium N-(1,2-dicarboxyethyl)-N-octadecylsulfo-succinamate; diamyl ester of sodium sulfosuccinic acid, dihexyl ester of sodium sulfosuccinic acid; dioctyl esters of sodium sulfosuccinic acid.

Other suitable anionic detergents utilizable herein are olefin sulfonates having about 12 to about 24 carbon atoms. The term "olefin sulfonates" is used herein to mean compounds which can be produced by the sulfonation of ∝-olefins by means of uncomplexed sulfur trioxide, followed by neutralization of the acid reaction mixture in conditions such that any sultones which have been formed in the reaction are hydrolyzed to give the corresponding hydroxy-alkanesulfonates. The sulfur trioxide can be liquid or gaseous, and is usually, but not necessarily, diluted by inert diluents, for example by liquid $SO_2$, chlorinated hydrocarbons, etc., when used in the liquid form, or by air, nitrogen, gaseous $SO_2$, etc., when used in the gaseous form.

The ∝-olefins from which the olefin sulfonates are derived are mono-olefins having 12 to 24 carbon atoms, preferably 14 to 16 carbon atoms. Preferably, they are straight chain olefins. Examples of suitable 1-olefins include 1-dodecene; 1-tetradecene; 1-hexadecene; 1-octadecene; 1-cicosene and 1-tetraeosene.

In addition to the true alkene sulfonates and a proportion of hydroxy-alkanesulfonates, the olefin sulfonates can contain minor amounts of other materials, such as alkene disulfonates depending upon the reaction conditions, proportion of reactants, the nature of the starting olefins and impurities in the olefin stock and side reactions during the sulfonation process.

A specific ∝-olefin sulfonate mixture of the above type is described more fully in US-A-3,332,880.

Another class of anionic organic detergents are the $\beta$-alkyloxy alkane sulfonates. These compounds have the following formula:

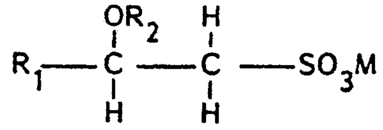

where $R_1$ is a straight chain alkyl group having from 6 to 20 carbon atoms, $R_2$ is a lower alkyl group having from 1 (preferred) to 3 carbon atoms, and M is a water-soluble cation as hereinbefore described.

Specific examples of β-alkyloxy-alkane-1-sulfonates, or alternatively 2-alkyloxy-alkane-1-sulfonates, having low hardness (calcium ion) sensitivity useful herein to provide superior cleaning levels under household washing conditions include:

potassium-β-methoxydecanesulfonate, sodium 2-methoxytridecanesulfonate, potassium 2-ethoxytetradecylsulfonate, sodium 2-isopropoxyhexadecylsulfonate, lithium 2-t-butoxytetradecylsulfonate, sodium β-methoxyoctadecylsulfonate, and ammonium -n-propoxydodecylsulfonate.

Another suitable class of anionic surfactants are the water-soluble salts of the organic sulfuric acid reaction products of the general formula:

$$R_1—SO_3—M$$

wherein $R_1$ is chosen from the group consisting of a straight or branched chain, saturated aliphatic hydrocarbon radical having from 8 to 24, preferably 12 to 18, carbon atoms; and M is a cation. Important examples are the salts of an organic sulfuric acid reaction product of a hydrocarbon of the methane series, including iso-, neo-, ineso-, and n-paraffins, having 8 to 24 carbon atoms, preferably 12 to 18 carbon atoms and a sulfonating agent e.g., $SO_3$, $H_2SO_4$, oleum, obtained according to known sulfonation methods, including bleaching and hydrolysis. Preferred are alkali metal and ammonium sulfonated $C_{12-18}$- n-paraffins.

Many additional nonsoap synthetic anionic surfactants are described in McCUTCHEON'S, DETERGENTS AND EMULSIFIERS, 1984 ANNUAL, published by Allured Publishing Corporation. Also US-A-3,929,678 discloses many other anionic as well as other surfactant types.

The above-mentioned surfactants can be used alone or in combination in the shampoo compositions of the present invention. The alkyl sulfates, the ethoxylated alkyl sulfates and mixtures thereof are preferred for use herein.

Non-Volatile Silicone Fluid

The non-volatile silicone fluid may be either a polyalkyl siloxane, a polyaryl siloxane, a polyalkylaryl siloxane or a polyether siloxane copolymer and is present at a level of from 0.1% to 10.00% preferably from 0.5% to 5.0%. Mixtures of these fluids may also be used and are preferred in certain executions. The dispersed silicone particles should also be insoluble in the shampoo matrix. This is the meaning of "insoluble" as used hereinbefore and hereinafter.

The essentially non-volatile polyalkyl siloxanes that may be used include, for example, polydimethyl siloxanes with viscosities ranging from 5 to 600,000 $mm^2.s^{-1}$ (centistokes) at 25° C. These siloxanes are available, for example, from the General Electric Company as the Viscasil (RTM) series and from Dow Corning as the Dow Corning 200 series. The viscosity can be measured by means of a glass capillary viscometer as set forth in Dow Corning Corporate Test Method CTM0004, July,20, 1970. Preferably the viscosity ranges from 350 $mm^2.s^{-1}$ (centistokes) to 100,000 $mm^2.s^{-1}$ (centistokes).

The essentially non-volatile polyalkylaryl siloxanes that may be used include, for example, polymethylphenylsiloxanes having viscosities of 15 to 65 $mm^2.s^{-1}$ (centistokes) at 25° C. These siloxanes are available, for example, from the General Electric Company as SF 1075 methyl phenyl fluid or from Dow Corning as 556 Cosmetic Grade Fluid.

The essentially non-volatile polyether siloxane copolymer that may be used is, for example, a polypropylene oxide modified dimethylpolysiloxane (e.g., Dow Corning DC-1248) although ethylene oxide or mixtures of ethylene oxide and propylene oxide may also be used.

References disclosing suitable silicones include the previously mentioned US-A-2,826,551 , US-A-3,964,500 , US-A-4,364,837 and GB-A-O,849,433. Silicon Compounds distributed by Petrarch Systems, Inc., 1984, also provides a very good listing of suitable silicone materials. Materials found especially useful are silicone gums described by Petrarch and others. Two other references include US-A-4,152,416, and Noll, Walter, Chemistry and Technology of Silicones , New York: Academic Press 1968. These materials generally have a molecular weight of from about 200,000 to about 700,000.

Xanthan Gum

Xanthan gum is the agent used in the present compositions to suspend the silicone fluid. This

biosynthetic gum material is commercially available and is a heteropolysaccharide with a molecular weight of greater than 1 million. It contains D-glucose, D-mannose and D-glucuronate in the molar ratio of 2.8:2.0:2.0. The polysaccharide is partially acetylated with ~4.7% acetyl. This information and other is found in Whistler, Roy L. Editor Industrial Gums - Polysaccharides and Their Derivatives New York: Academic Press, 1973. Kelco, a Division of Merck & Co., Inc. offers xanthan gum as Keltrol (RTM). The gum is present at a level of from 0.4% to 5%, preferably from 0.4% to 3%, more preferably from 0.6% to 1.2% in the compositions of the present invention.

## Water

Water is the last essential component of the present invention and forms the remainder of the composition. It is generally present at a level of from 20% to 95%, preferably from 60% to 85%.

## Optional Components

The shampoos herein can contain a variety of nonessential optional components suitable for rendering such compositions more acceptable. Such conventional optional ingredients are well known to those skilled in the art, e.g., pearlescent aids such as ethylene glycol distearate; preservatives such as benzyl alcohol, methyl paraben, propyl paraben and imidazolidinyl urea; cationic surfactants such as cetyl trimethyl ammonium chloride, stearyldimethyl benzyl ammonium chloride, and di(partially hydrogenated tallow) dimethylammonium chloride; thickeners and viscosity modifiers such as a diethanolamide of a long chain fatty acid (e.g., PEG 3 lauramide), amine oxides, block polymers of ethylene oxide and propylene oxide such as Pluronic F88 offered by BASF Wyandotte, fatty alcohols such as cetearyl alcohol, sodium chloride, sodium sulfate, polyvinyl alcohol, and ethyl alcohol; pH adjusting agents such as citric acid, succinic acid, phosphoric acid, sodium hydroxide, sodium carbonate, etc.; perfumes; dyes; and, sequestering agents such as disodium ethylenediamine tetraacetate. Such agents generally are used individually at a level of from about 0.01% to about 10%, preferably from about 0.5% to about 5.0% by weight of the composition.

Another optional ingredient and one preferred for use in certain of the compositions of this invention, is a volatile silicone or a water insoluble hydrocarbon. These agents are disclosed in US-A-4,472,375 . These agents help disperse the higher molecular weight, non-volatile silicones in the product when the product is used. These agents are used at levels from 0.1% to 5%.

The pH of the present compositions is not critical and may be in the range of from 4 to about 10.

## METHOD OF MANUFACTURE

The shampoos of the present invention can be made in the following manner:

A) Mix the water and surfactant of the composition together using a turbine blade at 500 r.p.m.

B) Heat the mixture of A) to 66 °C, increase the agitation sufficient to create a vortex and disperse the xanthan gum in the vortex created. During this step the temperature is kept at about 66 °C.

C) Add the remaining ingredients (except for the silicone) while agitating at the same speed.

D) Add the silicone and shear the composition with a high shear mixer until the silicone particles are on average $\leq$ 10 µm in diameter (the particle size distribution may be from about 2 to about 55 µm.

E) Cool the composition to 27 °C while mixing at 500 r.p.m.

## INDUSTRIAL APPLICABILITY

The present compositions are used in a conventional manner for cleaning hair. From 0. 1g to 10g of a composition is applied to hair that has been wetted, generally with water, worked through the hair and then rinsed out.

The following Examples further describe and demonstrate the preferred embodiments within the scope of the present invention. The Examples are given solely for the purpose of illustration and are not to be construed as limitations of the present invention as many variations thereof are possible without departing from its scope.

## EXAMPLES I-V

The following compositions are representative of the present invention.

| Component | Weight % I | II | III | IV | V |
|---|---|---|---|---|---|
| $NH_4C_{12-14}$ Alkyl Sulfate | -- | 16.00 | 8.00 | 16.00 | 8.00 |
| $NH_4C_{12-14}$Alkyl (Ethoxy) Sulfate | -- | -- | 8.00 | -- | 8.00 |
| TEA $C_{12-14}$ Alkyl Sulfate 3 | 18.20 | -- | -- | -- | -- |
| Xanthan Gum | 0.75 | 0.75 | 0.40 | 2.00 | 0.75 |
| Cocamide MEA | 3.00 | 1.00 | 3.00 | 1.50 | 1.00 |
| 50% Caustic Soda | 0.60 | 0.01 | -- | -- | 0.01 |
| Citric Acid | -- | -- | 0.60 | -- | -- |
| Sodium Chloride | 1.00 | 0.12 | 1.50 | -- | -- |
| DC-200 (12,500 csk)[1] | 5.00 | -- | 3.00 | -- | -- |
| DC-200 (350 csk)[2] | -- | 1.00 | -- | -- | 2.00 |
| DC-200 (600,000 csk)[3] | -- | -- | -- | 3.00 | -- |
| Dye Solution | 0.15 | 0.65 | 0.15 | -- | 0.65 |
| Ethylene glycol distearate | -- | 0.75 | -- | -- | 0.75 |
| Preservative | 0.033 | 0.033 | 0.033 | -- | 0.033 |
| Cetearyl Alcohol | -- | 1.00 | -- | -- | 1.00 |
| Perfume | 0.60 | 1.00 | 0.60 | 0.50 | 1.00 |
| Silicone Gum[4] | -- | 1.00 | -- | -- | 1.00 |
| Water (Double Reverse Osmosis) | qs 100% → | | | | |

1 Dimethylpolysiloxane offered by Dow Corning Corporation
2 Dimethylpolysiloxane offered by Dow Corning Corporation
3 Dimethylpolysiloxane offered by Dow Corning Corporation
4 Dimethylpolysiloxane gum offered by General Electric Company as Silicone Compound SE-76

The above compositions are stable and deliver good conditioning to hair that is washed with them. If in the compositions other anionic surfactants such as alpha olefin sulfonates or alkyl glyceryl sulfonates, are

used in place of those listed, similar results are obtained. Similarly if silicones of other viscosities and types, such as those listed on pages 6-8, are used in place of those listed, similar results are obtained.

## Claims

**1.** A shampoo composition comprising:
(a) from 5% to 50% by weight of synthetic anionic surfactant or a mixture of synthetic anionic surfactants;
(b) from 0.1% to 10% by weight of a dispersed, insoluble, non-volatile silicone or a mixture of dispersed, insoluble, non-volatile silicones; and
(c) water;
characterised in that the composition additionally comprises from 0.4% to 5% by weight of xanthan gum.

**2.** A shampoo composition according to Claim 1 wherein the surfactant or mixture of surfactants is selected from alkyl sulfates, ethoxylated alkyl sulfates, alpha olefin sulfonates, alkyl sulfonates and mixtures thereof.

**3.** A shampoo composition according to Claim 1 or 2 wherein the non-volatile silicone or mixture of non-volatile silicones has a viscosity of from 5 to 600,000 $mm^2.s^{-1}$ (centistokes) at 25 °C.

**4.** A shampoo composition according to any of Claims 1 to 3 wherein the surfactant or mixture of surfactants is selected from alkyl sulfates, ethoxylated alkyl sulfates and mixtures thereof.

**5.** A shampoo composition according to any of Claims 1 to 4 wherein the non-volatile silicone is a polydimethylsiloxane.

**6.** A shampoo composition according to Claim 5 wherein the polydimethylsiloxane has a viscosity of 350 $mm^2.s^{-1}$ (centistokes) at 25 °C.

**7.** A shampoo composition according to any of Claims 1 to 6 wherein an amide is also present in the compostions.

**8.** A shampoo composition according to any of Claims 1 to 7 wherein the surfactant is an alkyl sulfate, preferably an ammonium alkyl sulfate.

**9.** A shampoo composition according to any of Claims 1 to 8 comprising a mixture of non-volatile silicones wherein one of the non-volatile silicones is a silicone gum.

**10.** A shampoo composition according to Claim 9 which additionally contains a volatile silicone.

**11.** A method of shampooing the hair comprising applying from 0.1g to 10g of a composition according to any of Claims 1 to 10 to the hair that has been wet with water and then rinsed out.

## Revendications

**1.** Composition de shampooing,comprenant :
(a) de 5 % à 50 % en poids d'un surfactif anionique synthétitique ou d'un mélange de surfactifs anioniques synthétiques ;
(b) de 0,1 % à 10 % en poids d'une silicone non volatile , insoluble , dispersée ou d'un mélange de silicones non-volatiles , insolubles , dispersées; et
(c) de l'eau ;
caractérisée en ce qu'elle comprend en outre de 0,4 % à 5 % en poids de gomme xanthane .

**2.** Composition de shampooing selon la revendication 1 , dans laquelle le surfactif ou le mélange de

surfactifs est choisi parmi des alkyl sulfates , des alkyl sulfates éthoxylés , des alpha oléfines sulfonates , des alkyl sulfonates et leurs mélanges .

3. Composition de shampooing selon la revendication 1 ou 2 , dans laquelle la silicone non volatile ou le mélange de silicones non volatiles a une viscosité de 5 à 600 000 $mm^2.s^{-1}$ (centististokes) à 25 °C.

4. Composition de shampooing selon l'une quelconque des revendications 1 à 3 , dans laquelle le surfactif ou le mélange de surfactifs est choisi parmi des alkyl sulfates , des alkyl sulfates éthoxylés et leurs mélanges .

5. Composition de shampooing selon l'une quelconque des revendications 1 à 4 , dans laquelle la silicone non volatile est un polydiméthylsiloxane .

6. Composition de shampooing selon la revendication 5 ,dans laquelle le polydiméthylsiloxane a une viscosité de 350 $mm^2.s^{-1}$ (centistokes) à 25 °C.

7. Composition de shampooing selon l'une quelconque des revendications 1 à 6 , dans laquelle un amide est également présent dans la composition .

8. Composition de shampooing selon l'une quelconque des revendications 1 à 7 , dans laquelle le surfactif est un alkyl sulfate , de préférence un alkyl sulfate d'ammonium .

9. Composition de shampooing selon l'une quelconque des revendications 1 à 8 , comprenant un mélange de silicones non volatiles,dans lequel l'une des silicones non volatiles est une gomme de silicone .

10. Composition de shampooing selon la revendication 9,qui con tient en outre une silicone volatile .

11. Procédé pour shampooingner la chevelure , comprenant l'application de 0,1 g à 10 g d'une composition selon l'une quelconque des revendications 1 à 10 à la chevelure qui a été mouillée à l'eau ,puis rincée .

**Ansprüche**

1. Shampoozusammensetzung, umfassend:
   (a) von 5 Gew.-% bis 50 Gew.-% an einem synthetischen anionischen grenzflächenaktiven Mittel oder einem Gemisch aus synthetischen anionischen grenzflächenaktiven Mitteln;
   (b) von 0,1 Gew.-% bis 10 Gew.-% an einem dispergierten, unlöslichen, nicht-flüchtigen Silikon oder einem Gemisch aus dispergierten, unlöslichen, nicht-flüchtigen Silikonen; und
   (c) Wasser;
   dadurch gekennzeichnet, daß die Zusammensetzung zusätzlich 0,4 Gew.-% bis 5 Gew.-% an Xanthangummi enthält.

2. Shampoozusammensetzung nach Anspruch 1, worin das grenzflächenaktive Mittel oder das Gemisch der grenzflächenaktiven Mittel aus Alkylsulfaten, ethoxylierten Alkylsulfaten, alpha-Olefinsulfonaten, Alkylsulfonaten und Gemischen hievon ausgewählt ist.

3. Shampoozusammensetzung nach Anspruch 1 oder 2, worin das nicht-flüchtige Silikon oder das Gemisch aus nicht-flüchtigen Silikonen bei 25° C eine Viskosität von 5 bis 600.000 $mm^2.s^{-1}$ - (Centistokes) besitzt.

4. Shampoozusammensetzung nach einem der Ansprüche 1 bis 3, worin das grenzflächenaktive Mittel oder das Gemisch aus grenzflächenaktiven Mitteln unter Alkylsulfaten, ethoxylierten Alkylsulfaten und Gemischen hievon ausgewählt ist.

5. Shampoozusammensetzung nach einem der Ansprüche 1 bis 4, worin das nicht-flüchtige Silikon ein Polydimethylsiloxan ist.

6. Shampoozusammensetzung nach Anspruch 5, worin das Polydimethylsiloxan bei 25° C eine Viskosität von 350 $mm^2.s^{-1}$ (Centistokes) besitzt.

7. Shampoozusammensetzung nach einem der Ansprüche 1 bis 6, worin in den Zusammensetzungen auch ein Amid vorliegt.

8. Shampoozusammensetzung nach einem der Ansprüche 1 bis 7, worin das grenzflächenaktive Mittel ein Alkylsulfat, vorzugsweise ein Ammoniumalkylsulfat ist.

9. Shampoozusammensetzung nach einem der Ansprüche 1 bis 8, umfassend ein Gemisch aus nicht-flüchtigen Silikonen, worin eines der nicht-flüchtigen Silikone ein Silikonkautschuk ist.

10. Shampoozusammensetzung nach Anspruch 9, welche zusätzlich ein flüchtiges Silikon enthält.

11. Ein Verfahren zum Shampoonieren von Haar, umfassend das Aufbringen von 0,1 g bis 10 g einer Zusammensetzung nach einem der Ansprüche 1 bis 10 auf das Haar, welches mit Wasser befeuchtet wurde, und das anschließende Ausspülen.